# EUROPEAN PATENT SPECIFICATION

(11) **EP 2 577 605 B1**
(45) Date of publication and mention of the grant of the patent: **04.05.2016**
(21) Application number: 11729339.9
(22) Date of filing: 19.05.2011
(51) Int. Cl.: G06T 7/00

(54) **AUTOMATED QUANTIFICATION OF INTRAVASCULAR EMBOLIZATION SUCCESS**
AUTOMATISIERTE QUANTIFIZIERUNG VON INTRAVASKULÄREN EMBOLISIERUNGSERFOLGEN
QUANTIFICATION AUTOMATIQUE DU SUCCÈS D'EMBOLISATION INTRAVASCULAIRE

(30) Priority: 02.06.2010 US 350526 P
(43) Date of publication of application: 10.04.2013
(73) Proprietor: Koninklijke Philips N.V., 5656 AE Eindhoven (NL); Philips Intellectual Property & Standards GmbH, 20099 Hamburg (DE)
(72) Inventor: WIESNER, Steffen Gunther, NL-5656 AE Eindhoven (NL); BERTRAM, Matthias, NL-5656 AE Eindhoven (NL); LIN, Ming De, NL-5656 AE Eindhoven (NL)
(74) Representative: van Velzen, Maaike Mathilde
(86) International application number: PCT/IB2011/052194
(87) International publication number: WO 2011/151752

(56) References cited:
- US-A1- 2009 116 711
- SUSANNE BONEKAMP; ATILLA PETER KIRALY; MEHMET AKIF GULSUN; PRASHANT JOLEPALEM; IHAB R. KAMEL: "First Experiences with a Novel Software Prototype for Assessment of Treatment Response of Liver Tumors to Transarterial Chemoembolization", SIEMENS MAGNETON FLASH, THE MAGAZINE OF MR, no. 1/2010, 1 May 2010 (2010-05-01), pages 47-58, XP000002658114,
- GULSUN M A ET AL: "MR ONCO-TREAT: A new tool for volumetric and functional analysis of hepatic tumors monitored with multi-modal MRI", 17TH ANNUAL ISMRM SCIENTIFIC MEETING AND EXHIBITION 2009 : HONOLULU, HAWAII, USA, 18 - 24 APRIL 2009, , 1 April 2009 (2009-04-01), page 2876, XP009151700, ISBN: 978-1-61567-338-4 Retrieved from the Internet: URL:http://cds.ismrm.org/protected/09MProc eedings/files/02876.pdf [retrieved on 2011-09-02]
- CUENOD C A ET AL: "Tumor angiogenesis: pathophysiology and implications for contrast-enhanced MRI and CT assessment", ABDOMINAL IMAGING, SPRINGER-VERLAG, NE, vol. 31, no. 2, 1 April 2006 (2006-04-01), pages 188-193, XP019364268, ISSN: 1432-0509
- Dushyant Sahani: "Perfusion CT: An Overview Of Technique And Clinical Applications", Proc. Intl. Soc. Mag. Reson. Med. 18, 1 May 2010 (2010-05-01), pages 1-12, XP055149377, ISBN: 978-1-61-782008-3
- Zuxing Kan ET AL: "Functional CT Quantification of Tumor Perfusion after Transhepatic Arterial Embolization in a Rat Model 1", Radiology, vol. 237, no. 1, 1 October 2005 (2005-10-01), pages 144-150, XP055149376, ISSN: 0033-8419, DOI: 10.1148/radiol.2371040526

## Description

### FIELD OF THE INVENTION

The invention relates to imaging systems which can be used for obtaining image data of tissues. In particular, the invention relates to a device and a method for automatically quantifying intravascular embolization success and to an imaging system capable of conducting such a method.

### BACKGROUND OF THE INVENTION

Transcatheter targeted chemoembolic drug delivery today represents the mainstay treatment of liver cancer, requiring intensive support by X-ray imaging with catheterization laboratory systems. Performing and analyzing a dual phase scan prior to an actual treatment, which dual phase scan comprises imaging during both the early-arterial and late-arterial phases of enhancement under utilization of an intra-arterially injected contrast agent for enhancement of vessels or tissue, e.g. tumorous tissue, the information from the dual phases allows visualization of e.g. a tumor-feeding vessel tree and a tumor itself. This may thereby facilitate identifying and reaching an optimal point of drug delivery and provides insight into the tumor dynamics.

While transcatheter arterial chemoembolization (also known as "TACE") using lipiodol is currently the treatment of choice for palliative management of unresectable liver cancer, more recent techniques using drug-eluting beads or radioactive microspheres as the injected embolizing material are becoming increasingly popular, promising to yield higher treatment success while reducing side effects to the patient and thus shortening the required length of stay in the hospital. Such a drug-eluting bead based treatment may become more important than TACE as the prevalent treatment of the future.

However, in these methods of treatment, the technical success of the intervention cannot be verified as simple as in a traditional lipiodol TACE treatment, because neither the drug-eluting beads nor the radioactive microspheres are radio-opaque and so the drug delivery cannot readily be monitored in all situations as is done during and after a lipiodol-based TACE treatment. The necessity to track the positions where beads are delivered is especially important for measuring the treatment efficacy. Further, it is mandatory to consider that any bead reflux or washout from a tumor site may produce very severe side-effects.

In "First Experiences with a Novel Software Prototype for Assessment of Treatment Response of Liver Tumours to Transarterial Chemoembolization", Siemens Magnetation Flash, no. 01/2010, pages 47-58 and "MR ONCO-TREAT: A New Tool for Volumetric and Functional Analysis of Hepatic Tumours Monitored with Multi-Modal MRI", 17^{th} Annual ISMRM Scientific Meeting and exhibition 2009, page 2876, MRI related methods for assisting radiologists for evaluating cancer treatment is disclosed.

### SUMMARY OF THE INVENTION

A judgement of success of treatment based on visual comparison of pre- and post- interventional CT or dual phase scans alone may only provide a subjective and qualitative measure. Furthermore, this approach of outcome control is complicated by the general difficulty in defining e.g. an exact outline of a tumor, by the occurring patient movement between pre- and post-treatment scans, and by the different appearance of the untreated and embolized tumors.

In particular, identifying the exact outline of a tumor is usually more difficult in the post-interventional scan due to its embolized state and the consequent under-perfusion. These issues make it difficult to relate and compare corresponding tumor regions within images before & after the treatment.

It would therefore be advantageous to achieve an automated and reliable quantification of intravascular embolization success, e.g. for use in imaging systems. It would be advantageous to provide a reliable and precise method for measuring the treatment efficacy of a transarterial chemoembolization treatment as described above.

To better address one or more of these concerns, in a first aspect of the invention there is provided an imaging system comprising a radiation source and a radiation detection module comprising a device for automatically quantifying intravascular embolization success.

The device comprises a registration unit adapted for registering a first image and a second image, wherein the first image is preferably obtained at a late-arterial phase before an intravascular embolization treatment and wherein the second image is preferably obtained at a late arterial phase after an intravascular embolization of a region of interest of a tissue to be examined. Registering the first and second images compensates shape deviations in the images induced by a patient and/or organ movement occurring between obtaining the two images. Preferably, the registering process is automatic.

The device further comprises a segmentation unit adapted for segmenting a tissue in the first image and in the second image, wherein the segmentation unit is configured to conduct a segmentation process only with the first image and the already determined segmentation borders are applied to the second image. Thus, a direct comparison between a pre- and a post-treatment phase of a tissue can be accomplished. Due to the prior registration of these images, the segmentation only needs to be performed on one image, that is, on the pre-treatment image, which usually offers a better visibility of the tissue of interest. The segmentation is then also used for the secondly acquired image. As proposed above, the registration is performed prior to the segmentation, which itself is only performed on one of the images. It is to be noted that the above features are not only directed to tumor tissues. In fact, intravascular embolization of a tumor tissue is a relevant application. Nevertheless, the above features also lead to an improved outcome control also for any other tissue structure of interest.

Further, the device comprises an evaluation unit for evaluating a perfusion of the tissue based on comparing the first image and the second image. The observed image enhancement within the tissue resulting from a contrast agent injected during the image acquisition, wherein the enhancement may be measured in Hounsfield Units ("HU"), may then quantitatively be assessed and compared between the pre- and post- interventional images and/or with the respective healthy tissue. This may result in a multitude of measures for an embolization success. Most of the conceivable embodiments of this invention differ mainly *in* the *type* of measure derived.

It is noted, that the first image and the second image are obtained under utilization of a contrast agent for enhancement of vessels and tissue of interest. Basically, the device according to the invention may be suitable for any image acquisition apparatus that is capable of obtaining an image of tissues of interest that are enhanced in comparison to healthy tissue due to the utilization of contrast agent uptake in a way that is different between pre- and post-intervention scans.

The gist of the invention lies in acquiring pre- and post-interventional images, e.g. by a CT or a dual phase scan under utilization of a contrast agent to enhancement of vessels and tissues and conducting the further steps of registering two respective late arterial phase images, segmenting the tissue and quantitatively evaluating and comparing the perfusion of the tissue before and after the treatment.

According to another aspect of the invention, the evaluation conducted by the evaluation unit may be based on the number of voxels inside a tissue region which show HU-values above a given threshold and may therefore be considered as being perfused. The pre- and post-treatment difference in perfused tissue volumes is a direct measure of the embolization success. The given threshold value may be derived from a healthy tissue nearby the segmented tissue or alternatively, a fixed patient-independent threshold adjusted to the imaging setup and protocol may be used. A reference region may be defined automatically by the evaluation unit, the segmentation unit or the registration unit. This may also be conducted semi-automatically or manually. Preferably, the HU-values are absolute or relative HU-enhancement values.

Obtained information about perfusion deviations may be visualized in a color-coded fashion on a screen and may also comprise calculated volume fractions.

According to yet another aspect a method for automatically quantifying intravascular embolization success is provided. The method according to the invention basically comprises the steps of obtaining a first (pre-interventional) image of a region of interest of a tissue, obtaining a second (post-interventional) image of the region of interest of the tissue, registering the first image and second image, segmenting a tissue of interest in the first image and in the second image including conducting a segmentation process only with the first image and applying the already determined segmentation borders to the second image, and evaluating the perfusion of the tissue based on comparing the first image and the second image. In particular, the evaluating includes determining a difference between the first image and the second image in a number of voxels within the tissue showing HU-values about a given threshold.

For improving the results of the method according to the invention the first image and the second image are obtained in a late arterial phase each, hence increasing the risibility of the tissue of interest.

According to still another aspect of the invention, the segmented tissue regions of the first image and of the second image are compared with a healthy reference tissue.

In another exemplary embodiment of the present invention a computer program or a computer program element is provided, which computer program element is a part of a computer program adapted for controlling a device, e.g. an imaging apparatus, according to one of the above described aspects, which, when being executed by a processing unit, is adapted to perform corresponding method steps according to the invention.

The computer program element may therefore be stored on a computing unit, a calculating device, an electronic device, which may also be part of an embodiment of the present invention. The computing unit may be adapted to perform or initiate a performing of method steps associated with the above described device. Moreover, it may be adapted to operate the components of the above described device. A computer program element may be loaded into a working memory of a data processor, which data processor may thus be equipped to carry out the method according to the invention.

This exemplary embodiment of the invention covers both, a computer program element that is adapted for using the invention right from the start and a computer program element that is adapted for using the invention through being integrated by means of an update to turn an existing program into a program that uses the invention.

Further, the computer program element may also be able to provide all necessary steps to fulfill the procedure of an exemplary embodiment of the method according to the invention as described above.

According to a further exemplary embodiment of the present invention, a computer readable medium, such as a CD-ROM or the like, is presented wherein the computer readable medium has a computer program element stored on it, which computer program element is described by the preceding section.

However, the computer program element may also be presented over a network like the World Wide Web and may be downloadable into the working memory of a data processor from such a network.

According to a further exemplary embodiment of the present invention, a medium for making a computer program element available for downloading is provided, which computer program element is adapted to perform a method according to one of the previously described embodiments of the invention.

Additional embodiments deriving from additional quantitative measures of tissue perfusion/embolization extracted from the registered and segmented images are conceivable and are understood to be included in the invention described in this disclosure.

Furthermore, it is to be noted that the outcome control of the treatment could be performed offline and outside of the intervention room.

The described functionality for quantitative outcome control is potentially relevant not only for all kinds of targeted drug delivery but also for other radiological interventions (e.g. uterine fibroid or uterine artery embolization, embolization of GI bleedings) that are or may be performed in a catheter laboratory using dual phase scans, CT imaging or any modality that utilizes a contrast agent for enhancement of a tissue in such a way that the enhancement is different before and after the treatment.

It has to be noted that exemplary embodiments of the invention are described with reference to different subject matters. In particular, some exemplary embodiments are described with reference to apparatus type claims whereas other exemplary embodiments are described with reference to method type claims. However, a person skilled in the art will gather from the above and the following description that, unless otherwise notified, in addition to any combination of features belonging to one type of subject matter also any combination between features relating to different subject matters, in particular between features of the apparatus type claims and features of the method type claims, is considered to be disclosed with this application. However, all features can be combined providing synergetic effects that are more than the simple summation of the features.

### BRIEF DESCRIPTION OF THE DRAWINGS

The aspects defined above and further aspects, features and advantages of the present invention can also be derived from the examples of embodiments to be described herein after and are explained with reference to examples of embodiments, but to which the invention is not limited. The invention will be described in more detail hereinafter with reference to the drawings.
- Fig. 1: shows a schematic overview of a device according to the invention.
- Fig 2a and 2b: show late arterial phases of dual phase scans taken before (2a) and after (2b) treatment with drug-eluting beads.
- Fig. 3a and 3b: show extracted tumor regions by means of segmentation applied to the pre-interventional image.
- Fig. 4a and 4b: show pre- and post-intervention enhanced tumor regions normalized by taking the difference to the mean value of the corresponding reference region.
- Fig. 5: shows a method according to the present invention in a block diagram.
- Fig. 6: is a block diagram of an imaging system according to the present invention.

### DETAILED DESCRIPTION OF EMBODIMENTS

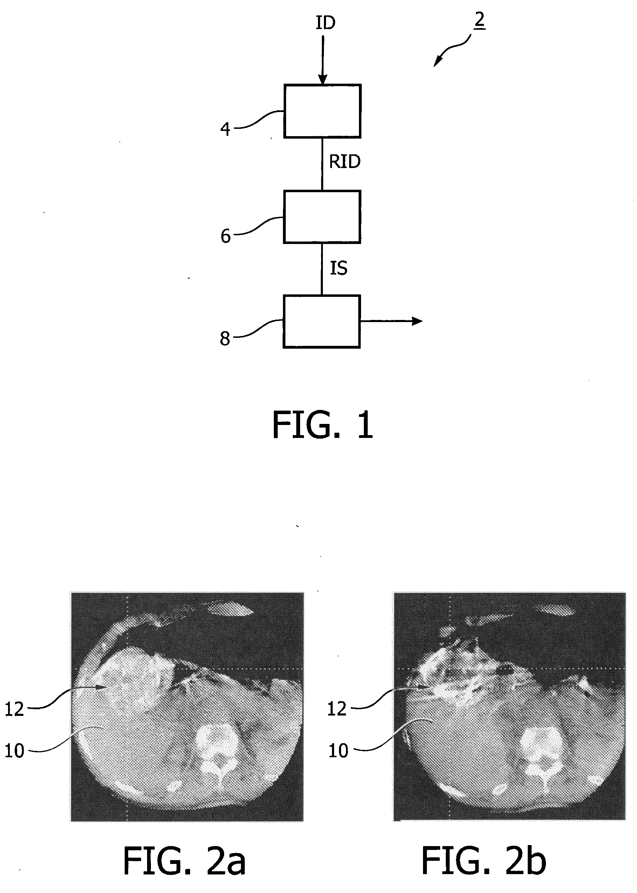

A first exemplary embodiment will be described in the following. Fig. 1 schematically shows a device 2 for automatically quantifying intravascular embolization success according to the first embodiment of the invention. The device 2 comprises a registration unit 4 which is provided with image data ID. This image data comprises a first image obtained before an intravascular embolization treatment is conducted and a second image obtained after an intravascular embolization treatment is conducted. The registration unit 4 registers the first image and the second image in order to compensate for patient/organ motion occurring between the acquisition time of the first image and the second image and provides registered image data RID. Registering itself may include selecting one or more reference points of the first image and of the second image and aligning these reference points relative to each other, thereby turning and stretching at least one of the first image and the second image. The original image data ID may be provided by an imaging system not shown in Fig. 1.

The device 2 further comprises a segmentation unit 6 which is adapted for segmenting a tissue of interest in the first image and in the second image provided by the registration unit 4, e.g. using well known segmentation methods. A first segmentation process is conducted with the first image only and then used also for the second image, since the first image and the second image are fully aligned by the registration process and the first image may provide a better visibility of the tissue of interest, e.g. a tumorous tissue. In particular, this involves applying segmentation borders, which were already determined in the first image, to the second image.

The device 2 further comprises an evaluation unit 8, which is provided with segments IS of the first image and the second image by the segmentation unit 6 containing tissue image parts of the first image and of the second image, e.g. tumorous image parts. The evaluation unit 8 is adapted for comparing these segments and thereby allowing to evaluate a perfusion of the tissue of interest. Comparing may include counting the number of voxels in each of the image segments data showing HU-values above a given threshold and then compare. The voxels that reach above a given threshold may be considered as perfused. Perfused areas may be shown in different colors on a screen attached to the device 2 according to the invention.

As an example for a tissue of interest in fig. 2a a tissue 10 with a tumorous region 12 is depicted before a treatment with drug-eluting beads is conducted. Therefore, the image shown in fig. 2a will be referred to as "first image". The first image is obtained at a late arterial phase, which may begin a short duration after a contrast medium has been transmitted and provides a best contrast between the tumorous region 12 and normal tissue.

In fig. 2b the tissue 10 is depicted after the treatment with drug-eluting beads has been conducted and is therefore referred to as "second image". The first image and second image are registered and it is clearly visible that the enhancement within the tumor region 12 in fig. 2a and 2b differ.

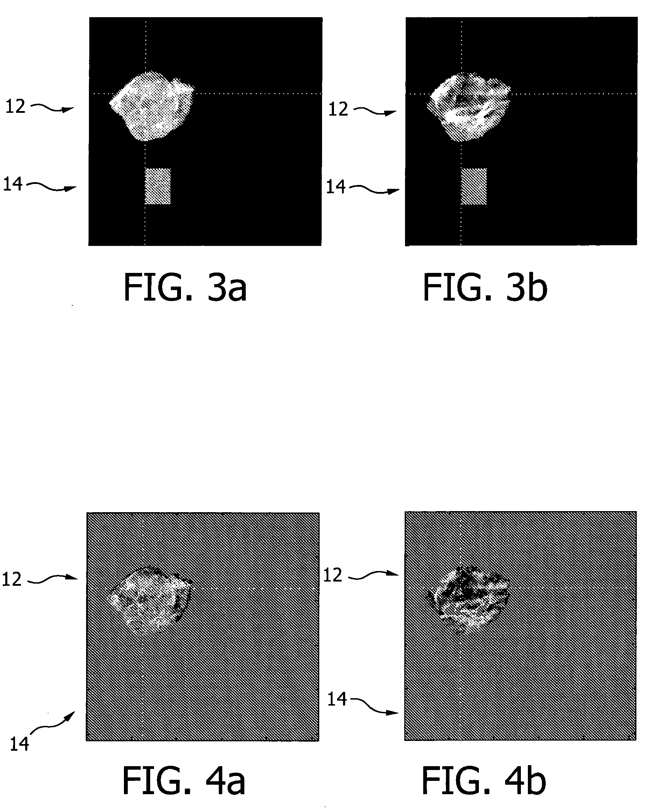

For a better ability to judge the treatment success of the intravascular embolization a segmentation of the tumor region 12 is conducted, as can be seen in fig. 3a and

3b. Such a segmentation is directed to partitioning image data into contiguous regions representing individual anatomical objects and is usually a prerequisite for further investigations. Segmentation may be conducted manually in a rather time-consuming process, or by an automatic process. In either way the segmentation process depends on the certain kind of examined tissue and may comprise different methods already known to a person skilled in the art. Therefore, the segmentation process is not described in detail.

A measure to judge the treatment success can be obtained by comparing the tumor regions 12 from the figs. 3a and 3b since the tumor region in fig. 3b is less perfused than the tumor region 12 of fig. 3a. The comparison may be achieved by different method steps already mentioned in the description part for fig. 1. If the comparison method includes comparing HU-values of the tumor region 12 with a predetermined HU-value or HU-interval, a reference section 14 that is outside the tumor region 12 may be defined in the first image and/or the second image, as indicated in figs. 3a and 3b. These reference regions comprise a mean HU-value.

For directly obtaining information about tissue regions being hypo- or hyper-perfused the image sections containing the tumor region 12 may be normalized, e.g. conducting a histogram normalization by taking the difference to the mean value of the corresponding reference region. In figs. 4a and 4b the normalized pre- and post-interventional segmented images are shown and it is clearly visible that the background now has the same shading as the reference area 14, hence rendering the reference area invisible. Regions with a brighter shading can now be considered hyper-perfused, wherein regions with a darker shading represent hypo-perfusion.

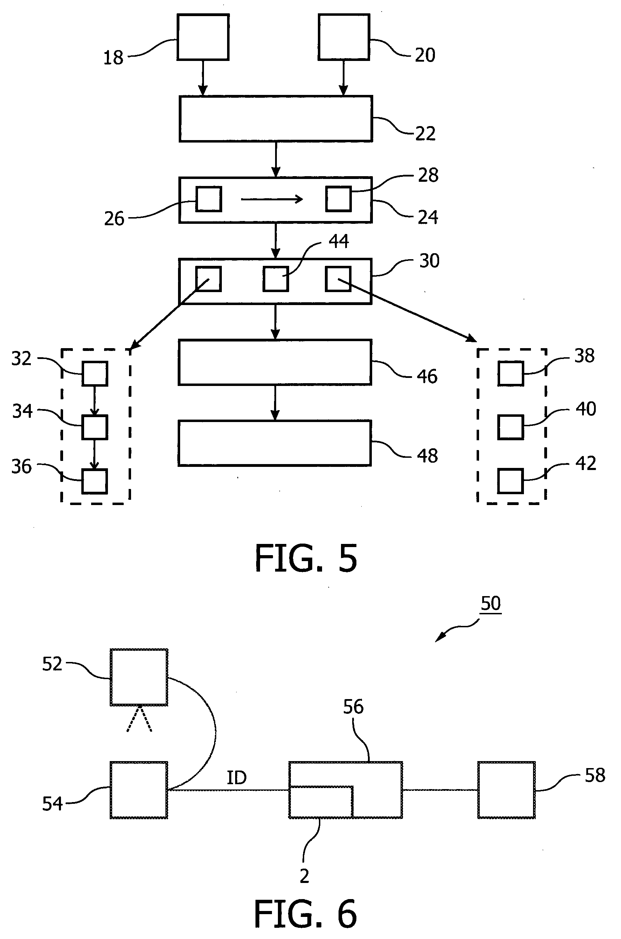

Fig. 5 shows a method for automatically quantifying intravascular embolization success according to the invention in a schematic block diagram. First of all, a first image of a region of interest of a tissue 10 is obtained 18. Later on, after the intravascular embolization has been conducted, a second image of the region of interest of the tissue is obtained 20. For enabling a comparison, the first image and second image are the registered 22. Afterwards, a tissue of interest in the first image and in the second image is segmented 24. The segmentation process is conducted for the first image only 26, since there the tissue of interest may comprise a better visibility. Then, the already determined segmentation borders are applied to the second image 28. Afterwards, the perfusion of the tissue of interest may then be evaluated 30 based on comparing the first image and the second image.

The evaluation comprises determining 32 a first number of voxels within the tissue in the first image comprising a HU-value above a given threshold, determining 34 a second number of voxels within the tissue in the second image comprising a HU-value above *a* given threshold and determining 36 the difference between the first number and the second number of voxels.

For improving the visibility of the evaluation, the differences in perfusion may be highlighted by visualizing 46 the difference through applying a suitable and unambiguous color-code.

Finally, the evaluated difference in perfusion may be output 48 to a screen or any other suitable output device.

An exemplary embodiment of an imaging system will be described in the following. Fig. 6 is a block diagram of an imaging system 50 according to the invention. The imaging system 50 comprises a radiation source 52, a radiation detection module 54, a data processing unit 56 with a central processing unit, a memory and a device 2 according to the first embodiment of the invention, described in regard to fig. 1.

Preferably the imaging system 50 further comprises a display unit 58, which display unit is connected with the data processing unit 56. The device 2 receives image data ID from the radiation detection module 54.

Merely as an example, but not limiting thereto, in Fig. 6 the radiation source 52 and the radiation detection module 54 are part of a C-arm system and the radiation source 52 therefore is an X-ray source and the radiation detection module is an X-ray detection module. The radiation source 52 and the radiation detection module 54 may also be part of a computer tomography system rotating around an object or subject to be examined.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage.

A computer program may be stored and/or distributed on a suitable medium, such as an optical storage medium or a solid state medium supplied together with or as part of other hardware, but may also be distributed in other forms, such as via the internet or other wired or wireless telecommunication systems.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive.

Any reference signs in the claims should not be construed as limiting the scope.

## Claims

1. An imaging system (50), comprising:
- a radiation source (52);
- a radiation detection module (54);
- a data processing unit (56) comprising a central processing unit, a memory, and a device (2) for automatically quantifying intravascular embolization success, the device comprising
- a registration unit (4) adapted for registering a first image and a second image;
- a segmentation unit (6) adapted for segmenting a tissue in the first image and in the second image, wherein the segmentation unit (6) is configured to conduct a segmentation process only with the first image and the determined segmentation borders are applied to the second image; and
- an evaluation unit (8) for evaluating a deviation of perfusion of the tissue by comparing the first image and the second image for quantitatively evaluating and comparing the perfusion of the tissue before and after the treatment,
the imaging system further comprising a display unit (58) connected with the data processing unit (56);
wherein the first image supplied to the device (2) is obtained before an intravascular embolization treatment and wherein the second image supplied to the device (2) is obtained after an intravascular embolization of a region of interest of a tissue to be examined,
wherein the device (2) receives image data (ID) from the radiation detection module (54), and
wherein the evaluation unit is configured to evaluate the deviation of perfusion of the tissue based on a difference between the first image and the second image in a number of voxels within the tissue showing HU-values above a given threshold.

2. The imaging system (50) according to claim 1,
wherein the HU-values are absolute or relative HU-enhancement values.

3. The imaging system (50) according to claim 1,
wherein the given threshold is derived from a healthy tissue nearby the segmented tissue.

4. The imaging system (50) according to claim 1,
wherein the given threshold is a fixed patient-independent threshold adjusted to an imaging setup and protocol.

5. The imaging system (50) according to claim 1,
wherein the tissue is a tumor tissue (12).

6. A method for automatically quantifying intravascular embolization success, comprising the steps:
- obtaining (18) a first image of a region of interest of a tissue (12) by means of a radiation source (52) and a radiation detection module (54);
- obtaining (20) a second image of the region of interest of the tissue (12) by means of the radiation source (52) and the radiation detection module (54);
- registering (22) the first image and second image;
- segmenting (24) the tissue of interest in the first image and in the second image, including conducting a segmentation process only with the first image and applying the determined segmentation borders to the second image, and
- evaluating (30) the perfusion of the tissue based on comparing the first image and the second image for quantitatively evaluating and comparing the perfusion of the tissue before and after the treatment,
wherein the first image is obtained before an intravascular embolization treatment and wherein the second image is obtained after an intravascular embolization, and
wherein the evaluating includes determining a difference between the first image and the second image in a number of voxels within the tissue showing HU-values above a given threshold

7. The method according to claim 6,
wherein the first image and the second image are obtained in a late-arterial phase each.

8. The method according to one of the claims 6 to 7, wherein evaluating (30) includes
- comparing the segmented region of the first image and of the second image with a healthy reference tissue.

9. The method according to one of the claims 6 to 8, further comprising the step of visualizing (46) the difference.

10. The method according to one of the claims 6 to 9, wherein obtaining the image includes conducting a contrast enhanced scan.

11. A computer program element for controlling an imaging system (50) according to one of the claims 1 to 5, which, when being executed by a data processing unit (56), is adapted to perform the method steps of one of the claims 6 to 10.

12. A computer readable medium having stored the program element of claim 11.

## Patentansprüche

1. Bildgebungssystem (50), das Folgendes umfasst:
- eine Strahlungsquelle (52);
- ein Strahlungsdetektionsmodul (54);
- eine Datenverarbeitungseinheit (56) umfassend eine zentrale Verarbeitungseinheit, einen Speicher und eine Vorrichtung (2) zum automatischen Quantifizieren des Erfolgs der intravaskulären Embolisierung, wobei die Vorrichtung Folgendes umfasst:
- eine Registrierungseinheit (4), die dafür ausgelegt ist, ein erstes Bild und ein zweites Bild miteinander zu registrieren;
- eine Segmentierungseinheit (6), die dafür ausgelegt ist, ein Gewebe in dem ersten Bild und in dem zweiten Bild zu segmentieren, wobei die Segmentierungseinheit (6) konfiguriert ist, um einen Segmentierungsprozess nur mit dem ersten Bild durchzuführen und die ermittelten Segmentierungsgrenzen werden auf das zweite Bild angewendet; und
- eine Evaluierungseinheit (8) zum Evaluieren einer Abweichung der Gewebeperfusion durch Vergleichen des ersten Bilds und des zweiten Bilds zum quantitativen Evaluieren und Vergleichen der Gewebeperfusion vor und nach der Behandlung,
wobei das Bildgebungssystem weiterhin eine Anzeigeeinheit (58) umfasst, die mit der Datenverarbeitungseinheit (56) verbunden ist;
wobei das erste der Vorrichtung (2) zugeführte Bild vor einer intravaskulären Embolisierungsbehandlung erlangt wird und wobei das zweite der Vorrichtung (2) zugeführte Bild nach einer intravaskulären Embolisierung einer interessierenden Region eines zu untersuchenden Gewebes erlangt wird,
wobei die Vorrichtung (2) Bilddaten (ID) von dem
Strahlungsdetektionsmodul (54) empfängt, und
wobei die Evaluierungseinheit konfiguriert ist, um die Abweichung der Gewebeperfusion basierend auf einem Unterschied zwischen dem ersten Bild und dem zweiten Bild in einer Anzahl von Voxeln innerhalb des Gewebes zu evaluieren, die HU-Werte über einem gegebenen Schwellenwart aufweisen.

2. Bildgebungssystem (50) nach Anspruch 1,
wobei die HU-Werte absolute oder relative HU-Verstärkungswerte sind.

3. Bildgebungssystem (50) nach Anspruch 1,
wobei der gegebene Schwellenwert von einem gesunden Gewebe in der Nähe des segmentierten Gewebes abgeleitet wird.

4. Bildgebungssystem (50) nach Anspruch 1,
wobei der gegebene Schwellenwert ein fester, patientenunabhängiger Schwellenwert ist, der auf ein Bildgebungs-Setup und Protokoll angepasst wird.

5. Bildgebungssystem (50) nach Anspruch 1,
wobei das Gewebe ein Tumorgewebe (12) ist.

6. Verfahren zum automatischen Quantifizieren des Erfolgs einer intravaskulären Embolisierung, wobei das Verfahren die folgenden Schritte umfasst:
- Erlangen (18) eines ersten Bilds einer interessierenden Region eines Gewebes (12) mithilfe einer Strahlungsquelle (52) und eines Strahlungsdetektionsmoduls (54);
- Erlangen (20) eines zweiten Bilds der interessierenden Region des Gewebes (12) mithilfe der Strahlungsquelle (52) und des Strahlungsdetektionsmoduls (54);
- Registrieren (22) des ersten Bilds mit dem zweiten Bild;
- Segmentieren (24) des interessierenden Gewebes in dem ersten Bild und in dem zweiten Bild, umfassend das Durchführen eines Segmentierungsvorgangs nur mit dem ersten Bild und Anwenden der ermittelten Segmentierungsgrenzen auf das zweite Bild, und
- Evaluieren (30) der Gewebeperfusion basierend auf dem Vergleichen des ersten Bilds und des zweiten Bilds zum quantitativen Evaluieren und Vergleichen der Gewebeperfusion vor und nach der Behandlung,
wobei das erste Bild vor einer intravaskulären Embolisierungsbehandlung erlangt wird und wobei das zweite Bild nach einer intravaskulären Embolisierung erlangt wird, und
wobei das Evaluieren das Ermitteln eines Unterschieds zwischen dem ersten Bild und dem zweiten Bild in einer Anzahl von Voxeln innerhalb des Gewebes umfasst, die HU-Werte über einem gegebenen Schwellenwart aufweisen.

7. Verfahren nach Anspruch 6,
wobei das erste Bild und das zweite Bild jeweils in einer spät-arteriellen Phase erlangt werden.

8. Verfahren nach einem der Ansprüche 6 bis 7, wobei das Evaluieren (30) Folgendes umfasst:
- Vergleichen der segmentierten Region des ersten Bilds und des zweiten Bilds mit einem gesunden Referenzgewebe.

9. Verfahren nach einem der Ansprüche 6 bis 8, das weiterhin den Schritt des Visualisierens (46) des Unterschieds umfasst.

10. Verfahren nach einem der Ansprüche 6 bis 9, wobei das Erlangen des Bilds das Durchführen eines kontrastverstärkten Scans umfasst.

11. Computerprogrammelement zum Steuern eines Bildgebungssystems (50) nach einem der Ansprüche 1 bis 5, das, wenn es durch eine Datenverarbeitungseinheit (56) ausgeführt wird, dafür ausgelegt ist, die Verfahrensschritte nach einem der Ansprüche 6 bis 10 durchzuführen.

12. Computerlesbares Medium, auf dem das Programmelement nach Anspruch 11 gespeichert ist.

## Revendications

1. Système d'imagerie (50), comprenant :
- une source de rayonnement (52) ;
- un module de détection de rayonnement (54) ;
- une unité de traitement de données (56) comprenant une unité centrale, une mémoire et un dispositif (2) pour quantifier automatiquement un succès d'embolisation intravasculaire, le dispositif comprenant :
- une unité d'alignement (4) apte à aligner une première image et une deuxième image ;
- une unité de segmentation (6) apte à segmenter un tissu dans la première image et dans la deuxième image, dans lequel l'unité de segmentation (6) est configurée pour effectuer un processus de segmentation uniquement avec la première image et les bordures de segmentation déterminées sont appliquées à la deuxième image ; et
- une unité d'évaluation (8) pour évaluer un écart de perfusion du tissu par la comparaison de la première image et de la deuxième image pour l'évaluation quantitative et la comparaison de la perfusion du tissu avant et après le traitement,
le système d'imagerie comprenant en outre une unité d'affichage (58) reliée à l'unité de traitement de données (56) ;
dans lequel la première image fournie au dispositif (2) est obtenue avant un traitement d'embolisation intravasculaire et dans lequel la deuxième image fournie au dispositif (2) est obtenue après une embolisation intravasculaire d'une région d'intérêt d'un tissu à examiner,
dans lequel le dispositif (2) reçoit des données d'image (ID) provenant du module de détection de rayonnement (54), et
dans lequel l'unité d'évaluation est configurée pour évaluer l'écart de perfusion du tissu sur la base d'une différence entre la première image et la deuxième image dans un nombre de voxels à l'intérieur du tissu indiquant des valeurs HU supérieures à un seuil donné.

2. Système d'imagerie (50) selon la revendication 1,
dans lequel les valeurs HU sont des valeurs absolues ou relatives d'amélioration HU.

3. Système d'imagerie (50) selon la revendication 1,
dans lequel le seuil donné est dérivé d'un tissu sain à proximité du tissu segmenté.

4. Système d'imagerie (50) selon la revendication 1,
dans lequel le seuil donné est un seuil fixe indépendant d'un patient ajusté à une configuration et un protocole d'imagerie.

5. Système d'imagerie (50) selon la revendication 1,
dans lequel le tissu est un tissu de tumeur (12).

6. Procédé de quantification automatique d'un succès d'embolisation intravasculaire, comprenant les étapes de :
- l'obtention (18) d'une première image d'une région d'intérêt d'un tissu (12) au moyen d'une source de rayonnement (52) et d'un module de détection de rayonnement (54) ;
- l'obtention (20) d'une deuxième image de la région d'intérêt du tissu (12) au moyen de la source de rayonnement (52) et du module de détection de rayonnement (54) ;
- l'alignement (22) de la première image et de la deuxième image ;
- la segmentation (24) du tissu d'intérêt dans la première image et dans la deuxième image, comprenant l'exécution d'un processus de segmentation uniquement avec la première image et l'application des bordures de segmentation déterminées à la deuxième image ; et
- l'évaluation (30) de la perfusion du tissu sur la base de la comparaison de la première image et de la deuxième image pour l'évaluation quantitative et la comparaison de la perfusion du tissu avant et après le traitement,
dans lequel la première image est obtenue avant un traitement d'embolisation intravasculaire et dans lequel la deuxième image est obtenue après une embolisation intravasculaire, et
dans lequel l'évaluation comprend la détermination d'une différence entre la première image et la deuxième image dans un nombre de voxels à l'intérieur du tissu indiquant des valeurs HU supérieures à un seuil donné.

7. Procédé selon la revendication 6,
dans lequel chacune de la première image et la deuxième image est obtenue dans une phase artérielle tardive.

8. Procédé selon l'une des revendications 6 et 7, dans lequel l'évaluation (30) comprend :
- la comparaison de la région segmentée de la première image et de la deuxième image à un tissu de référence sain.

9. Procédé selon l'une des revendications 6 à 8, comprenant en outre l'étape de la visualisation (46) de la différence.

10. Procédé selon l'une des revendications 6 à 9, dans lequel l'obtention de l'image comprend l'exécution d'un balayage à contraste amélioré.

11. Élément de programme informatique pour commander un système d'imagerie (50) selon l'une des revendications 1 à 5 qui, lorsqu'il est exécuté par une unité de traitement de données (56), est apte à effectuer les étapes de procédé selon l'une des revendications 6 à 10.

12. Support lisible par ordinateur sur lequel est mémorisé l'élément de programme selon la revendication 11.
